# EUROPÄISCHE PATENTSCHRIFT

(11) **EP 2 872 113 B1**
(45) Veröffentlichungstag und Bekanntmachung des Hinweises auf die Patenterteilung: **04.04.2018**
(21) Anmeldenummer: 13729966.5
(22) Anmeldetag: 20.06.2013
(51) Int. Cl.: A61K 8/41, A61Q 5/06, A61K 8/65, A61K 8/81

(54) **KOSMETISCHES HITZESCHUTZMITTEL**
COSMETIC HEAT-PROTECTION AGENT
PRODUIT COSMÉTIQUE THERMOPROTECTEUR

(30) Priorität: 10.07.2012 DE 102012212010
(43) Veröffentlichungstag der Anmeldung: 20.05.2015
(73) Patentinhaber: Henkel AG & Co. KGaA, 40589 Düsseldorf (DE)
(72) Erfinder: KNAPPE, Thorsten, 22869 Schenefeld (DE); FLODROP VAN, Helga, 40221 Düsseldorf (DE)
(86) Internationale Anmeldenummer: PCT/EP2013/062912
(87) Internationale Veröffentlichungsnummer: WO 2014/009128

(56) Entgegenhaltungen:
- WO-A1-92/17153
- DE-A1- 19 738 303
- JP-A- S6 354 313
- US-A- 4 673 568
- US-A- 5 756 106
- US-B1- 6 521 219
- DATABASE GNPD [Online] MINTEL; Januar 2010 (2010-01), "Finishing Care", XP002721684, Database accession no. 1260848
- DATABASE GNPD [Online] MINTEL; März 2014 (2014-03), "220° Heat Protection Spray", XP002721685, Database accession no. 2342800

## Beschreibung

Die Anmeldung betrifft das technische Gebiet der temporären oder permanenten Verformung menschlicher Haare. Die Anmeldung betrifft insbesondere Hitzeschutzmittel für den Einsatz in Verfahren zur thermisch unterstützten temporären oder permanenten Umformung menschlicher Haare.

Für die permanente oder temporäre Formgebung menschlicher Haare werden eine Reihe unterschiedlicher Techniken, beispielsweise Gestaltungen wie Lockung, Glättung, Toupierung oder auch Festigung, eingesetzt. Diese Techniken beruhen auf dem Einsatz festigender kosmetischer Mittel, so genannter Stylingmittel, wie Haarsprays, Haarwachse, Haargele, Haarfestiger, Fönwellen, Stylingsprays.

Alternativ oder in Ergänzung zu der Behandlung mit festigenden kosmetischen Mitteln können die Haare einer thermischen Behandlung unterzogen werden.

Um glattes Haar zu wellen oder zu kräuseln, werden die Haare unter Wärmeeinwirkung, beispielsweise mit Hilfe eines Föhns mit Diffusor oder eines Lockenstabs, verformt. Dabei wird das glatte Haar um den aufgeheizten Lockenstab oder den Diffusor gewickelt, wobei wiederum Temperaturen bis hin zu 250°C erreicht werden. In diesem Verfahren kommen zur Verbesserung des Stylingergebnisses in der Regel Stylingsprays zum Einsatz, die vor der eigentlichen Temperaturbehandlung auf das Haar aufgebracht werden.

Zur Glättung der Haare werden spezielle Glätteisen eingesetzt. Glätteisen weisen zwei parallel angeordnete Metall- oder Keramikplatten auf, durch welche die Haare, nach Aufheizen der Platten, gezogen werden. Handelsübliche Glätteisen lassen sich auf Temperaturen im Bereich von 150-250 °C aufheizen. Ziel der Glätteisenanwendung ist es, gewelltes bis gelocktes Haar thermisch/physikalisch zu glätten. Sollen Haare durch ein Glätteisen geglättet werden, wird auf das Haar üblicherweise vorher als Stylingmittel für Glätteisen, ein Stylingspray, auch Glätteisenspray bezeichnet, aufgetragen. Das Spray unterstützt dabei das Gleiten des Eisens sowie die Glättung des Haares.

Aus dem Stand der Technik sind zahlreiche kosmetische Zusammensetzungen zur temporären Haarverformung auch zur Anwendung unter Wärmeeinwirkung bekannt. Die europäische Patentanmeldung EP 1 750 656 A1 beschreibt Hitzeschutzsprays, die neben weiteren Inhaltsstoffen kationische Weizenproteinhydrolysate enthalten.

Der Nachteil der aus dem Stand der Technik bekannten kosmetischen Zusammensetzungen zur temporären Haarverformung unter Wärmeeinwirkung ist deren intensive Geruchsbildung bei Wärmeanwendung, die beim Anwender einen leichten Hustenreiz während der Wärme-behandlung des Haares auslösen kann. Weiterhin ist insbesondere der durch diese Mittel erreichte Hitzeschutz des Haares verbesserungswürdig.

Es war somit Aufgabe der vorliegenden Erfindung, eine kosmetische Zusammensetzung zur Behandlung der Haare unter Wärmeeinwirkung bereitzustellen, die auch bei Temperaturen von 250°C stabil ist, bei diesen hohen Temperaturen keine gesundheitlich bedenklichen Substanzen freisetzt und bei einer Anwendung in diesem Temperaturbereich eine gegenüber herkömmlichen Zusammensetzungen deutlich reduzierte Geruchsbildung aufweist. Weiterhin sollte sich die kosmetische Zusammensetzung insbesondere bei Verformungsverfahren im Temperaturbereich oberhalb 150°C durch verbesserte Hitzeschutzeigenschaften auszeichnen, das Haar also besser als mit herkömmlichen Mitteln vor der schädigen Wirkung der eingesetzten hohen Temperaturen schützen.

Es wurde festgestellt, dass sich die zuvor beschriebenen technischen Aufgaben durch spezielle Hitzeschutzsprays auf Grundlage quaternisierten Collagenhydrolysate lösen lassen. Der Einsatz von Collagenhydrolysaten in Haarpflegemitteln wird in dem US-amerikanischen Patent US 4,374,125 und in der deutschen Patentanmeldung DE 41 09 999 A1 beschrieben.

Ein erster Gegenstand der vorliegenden Anmeldung ist daher eine kosmetische Zusammensetzung, enthaltend
a) mindestens eine erste quartäre Ammoniumverbingung aus der Gruppe der quaternisierten Collagenhydrolysate;
b) Cetyltrimethylammoniumchlorid;
c) mindestens ein filmbildendes Polymer aus der Gruppe der Vinylpyrrolidon/VinylacetatCopolymere
d) Wasser.

Das US-amerikanische Patent US 4,673,568 beschreibt eine Fönlotion, die quaternisiertes Collagenhydrolysat, eine weitere quartäre Ammoniumverbindung, ein PVPNA Copolymer und Wasser enthält.

Aus der deutschen Patentanmeldung DE 197 38 303 A1 sind haarkosmetische Sprühkuren bekannt, welche faserstrukturverbessernde Wirkstoffe wie Panthenol oder Collagenhydrolysat in Kombination mit konditionierenden Wirkstoffen wie Alkyltrimethylammoniumhalogeniden sowie filmbildende Polymere enthalten.

Die erfindungsgemäßen kosmetischen Zusammensetzungen enthalten als ersten wesentlichen Bestandteil eine quartäre Ammoniumverbingung a) aus der Gruppe der quaternisierten Collagenhydrolysate. In bevorzugten Zusammensetzungen beträgt der Gewichtsanteil der auch als kationische derivatisierten Collagenhydrolysate bezeichneten quartären Ammoniumverbindung a) am Gesamtgewicht der Zusammensetzung 0,05 bis 4,0 Gew.-%, bevorzugt 0,1 bis 2,0 Gew.-% und inbesondere 0,2 bis 1,0 Gew.-%.

Kationisch derivatisierte Collagenhydrolysate a) sind Substanzmischungen, die beispielsweise durch Umsetzung von alkalisch, sauer oder enzymatisch hydrolysierten Proteinen mit kationischen Alkylierungsmitteln erhalten werden. Solche Alkylierungsmittel sind z. B. die Glycidyltrialkylammoniumchloride oder die 3-Chlor-2-hydroxypropyl-trialkyl-ammoniumchloride. Bevorzugt enthalten diese Verbindungen drei Methylgruppen oder eine langkettige Alkyl-oder Acylamidopropyl- gruppe und zwei Methylgruppen in der quartären Ammoniumgruppe. Durch Kondensation mit den Proteinhydrolysaten werden dann die kationisch derivatisierten Collagenhydrolysate a) erhalten. Verbindungen, die eine lange Alkylkette enthalten, sind bevorzugt. Bevorzugte kationisch derivatisierte Collegenhydrolysate a) weisen eine N,N-Dimethyl-N-Cₐ₋₂₂ Alkyl-Ammoniumgruppe, vorzugsweise eine N,N-Dimethyl-N-C₈₋₁₆ Alkyl-Ammoniumgruppe auf. Bevorzugte kationisch derivatisierte Collegenhydrolysate a) sind insbesondere durch eine N, N-Dimethyl-N-kokosalkyl- Ammoniumgruppe gekennzeichnet. Besonders bevorzugte quaternisierte Collagenhydrolysate sind unter der INCI Bezeichnung Lauryldimonium Hydroxypropyl Hydrolyzed Collagen zusammengefasst.

Hinsichtlich ihrer kosmetischen Eigenschaften, insbesondere des Hitzeschutzes der kosmetischen Zusammensetzungen hast sich der Einsatz von quartären Ammoniumverdingungen a) mit einem Molekulargewicht oberhalb 1000 g/mol, vorzugsweise zwischen 1200 und 10000 g/mol, besonders bevorzugt zwischen 1500 und 5000 g/mol und insbesondere zwischen 2000 und 2800 g/mol als vorteilhaft erwiesen. Entsprechende Mittel werden erfindungsgemäß bevorzugt.

Als zweiten wesentlichen Bestandteil enthalten die erfindunsgemäßen Mittel Cetyltrimethylammoniumchlorid. Der Gewichtsanteil vom Cetyltrimethylammoniumchlorid am Gesamtgewicht der kosmetischen Zusammensetzung beträgt vorzugsweise 0,1 bis 6,0 Gew.-%, bevorzugt 0,2 bis 4,0 Gew.-% und inbesondere 0,4 bis 2,0 Gew.-%.

In Bezug auf die Hitzeschutzwirkung der erfindungsgemäßen kosmetischen Zusammensetzungen hat es sich als vorteilhaft erwiesen, wenn die quartäre Ammoniumverbindung b) ein Molekulargewicht unterhalb 1000 g/mol, vorzugsweise zwischen 200 und 800 g/mol, besonders bevorzugt zwischen 250 und 500 g/mol und insbesondere zwischen 300 und 400 g/mol aufweist.

Als quartäre Ammoniumverbindung b) wird Cetyltrimethylammoniumchlorid eingesetzt. Kosmetische Zusammensetzungen enthalten Cetyltrimethylammoniumchlorid. Der Gewichtsanteil der Alkyltrimethylammoniumhalogenide, vorzugsweise aus der Gruppe der Alkyltrimethylammoniumchloride, insbesondere des Cetyltrimethylammoniumchlorids am Gesamtgewicht der erfindungsgemäßen Zusammensetzung beträgt vorzugsweise 0,05 bis 4,0 Gew.-%, bevorzugt 0,1 bis 2,0 Gew.-% und inbesondere 0,2 bis 1,0 Gew.-%.

Zur Verbesserung der Produkteigenschaften der erfindungsgemäßen kosmetischen Zusammensetzungen hat es sich als vorteilhaft erwiesen, ein spezifisches Gewichtsverhältnis der quartären Ammoniumverbindungen a) und b) einzuhalten. Kosmetische Zusammensetzung, bei denen das Gewichtsverhältnis von quartärer Ammoniumverbingung a) zu Cetyltrimethylammoniumchlorid b) 5:1 bis 1:5, vorzugsweise 4:1 bis 1:4, besonders bevorzugt 3:1 bis 1:3 und insbesondere 2: 1 bis 1:2 beträgt, werden aus diesem Grund bevorzugt.

Die erfindungsgemäßen kosmetischen Zubereitungen enthalten als weiteren Bestandteil ein filmbildendes Polymer c). Der Gewichtsanteil dieses Polymers c) am Gesamtgewicht der kosmetischen Zubereitung beträgt vorzugsweise 0,5 bis 12 Gew.-%, bevorzugt 1,0 bis 10 Gew.-% und inbesondere 2,0 bis 8,0 Gew.-%.

Als filmbildende Polymere c) werden nichtionische Polymere eingesetzt.

Geeignete nichtionische Polymere sind beispielsweise:
- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere.

Erfindungsgemäß bevorzugt ist z.B. das wasserlösliche, filmbildende VinylpryrrolidonNinylacetat-Copolymer, erhältlich unter der Handelsbezeichnung PVPNA W-635 (INCI-Bezeichnung: VP/VA Copolymer).

Die erfindungsgemäßen Zusammensetzungen können temperaturstabile kationische Polymere enthalten. Unter kationischen Polymeren sind Polymere zu verstehen, welche in der Haupt- und/oder Seitenkette Gruppen aufweisen, welche "temporär" oder "permanent" kationisch sein können. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (III), 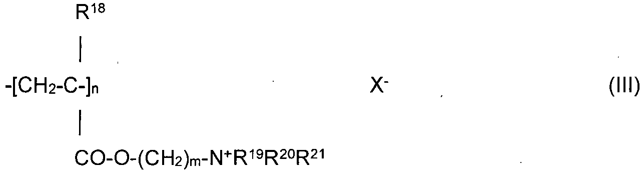 in der R¹⁸ = -H oder -CH₃ ist, R¹⁹, R²⁰ und R²¹ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (III) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹⁸ steht für eine Methylgruppe
- R¹⁹, R²⁰ und R²¹ stehen für Methylgruppen
- m hat den Wert 2.

Als physiologisch verträgliche Gegenionen X- kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gegebenenfalls vernetzte, Poly(methacryloyloxyethyltrimethylammoniumchlorid) mit der INCI-Bezeichnung Polyquaternium-37. Die Vernetzung kann, falls gewünscht, mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (III) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwässrige Polymerdispersion unter der Bezeichnung Salcare® SC 92 erhältlich.

Weitere bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400, sind bevorzugte quaternierte Cellulose-Derivate,
- kationische Alkylpolyglycoside gemäß der DE-PS 44 13 686,
- kationisierter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905, HM 552 und Hold angeboten werden.
- quaternierter Polyvinylalkohol,

Bevorzugte kationische Polymere sind weiterhin die unter den INCI-Bezeichnungen
- Polyquaternium 2,
- Polyquaternium 17,
- Polyquaternium 18 und
- Polyquaternium 27
bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind. Weitere erfindungsgemäße kationische Polymere sind die so genannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben. Besonders gut geeignete Chitosane weisen einen Deacetylierungsgrad von wenigstens 80 % und ein Molekulargewicht von 5 · 105 bis 5 · 106 (g/mol) auf.

Zur Herstellung erfindungsgemäßer Zubereitungen muss das Chitosan in die Salzform überführt werden. Dies kann durch Auflösen in verdünnten wässrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Zitronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

Bevorzugte kationische, filmbildende Polymere sind die Vinylpyrrolidon-Methoimidazoliniumchlorid-Copolymere, wie sie unter der Bezeichnung "Luviquat®" angeboten werden, z.B. Luviquat FC 370 (INCI-Bezeichnung: Polyquaternium-16), Luviquat Style (INCI-Bezeichnung: Polyquaternium-16) und Luviquat Hold (INCI-Bezeichnung: Polyquaternium-46), sowie das unter der INCI-Bezeichnung Polyquarternium-55 bekannte kationische Terpolymer aus Vinylpyrrolidon, Dimethylaminopropylmethacrylamid und Lauryldimethylpropylmethacrylamidoammoniumchlorid, welches unter der Bezeichnung Styleze W erhältlich ist.

Der Gewichtsanteil der Vinylpyrrolidon/Vinylacetat-Copolymere am Gesamtgewicht der kosmetischen Zubereitung beträgt vorzugsweise 0,5 bis 12 Gew.-%, bevorzugt 1,0 bis 10 Gew.-% und inbesondere 2,0 bis 8,0 Gew.-%.

Die erfindungsgemäßen Zusammensetzungen enthalten als Lösungsmittel Wasser Kosmetische Zusammensetzung, dadurch gekennzeichnet, dass sie bezogen auf ihr Gesamtgewicht 70 bis 99 Gew.-%, vorzugweise 75 bis 95 Gew.-% und insbesondere 80 bis 90 Gew.-% Wasser enthalten, werden erfindungsgemäß bevorzugt. Besonders bevorzugte Zusammensetzungen enthalten Wasser und Ethanol, wobei der Gewichtsanteil des Ethanols vorzugsweise deutlich unterhalb des Gewichtsanteils des Wassers liegt und bevorzugt 1,0 bis 10 Gew.-%, besonders bevorzugt 2,0 bis 8,0 Gew.-% und insbesondere 3,0 bis 6,0 Gew.-%, jeweils bezogen auf das Gesamtgewicht der Zusammensetzung, beträgt.

Der pH-Wert erfindungsgemäßer Zusammesetzungen beträgt vorzugsweise pH 4,0 bis 8,0, bevorzugt pH 5,0 bis 7,5 und insbesoner pH 5,5 bis 7,0.

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 31 | Formel 32 | Formel 33 | Formel 34 | Formel 35 |
|---|---|---|---|---|---|
| Quaternisiertes Collagenhydrolysat a) | 0,05 bis 4,0 | 0,1 bis 2,0 | 0,2 bis 1,0 | 0,3 | 1,0 |
| Cetyltrimethylammoniumchlorid | 0,1 bis 6,0 | 0,2 bis 4,0 | 0,4 bis 2,0 | 0,5 | 0,5 |
| Vinylpyrrolidon/Vinylacetat-Copolymer | 0,5 bis 12 | 1,0 bis 10 | 2,0 bis 8,0 | 5,0 | 2,0 |
| Wasser | 70 bis 99 | 75 bis 95 | 80 bis 90 | 87 | 85 |
| Misc | add 100 | add 100 | add 100 | add 100 | add 100 |

| | Formel 36 | Formel 37 | Formel 38 | Formel 39 | Formel 40 |
|---|---|---|---|---|---|
| Quaternisiertes Collagenhydrolysat a) MW: 2000 und 2800 g/mol | 0,05 bis 4,0 | 0,1 bis 2,0 | 0,2 bis 1,0 | 0,3 | 1,0 |
| Cetyltrimethylammoniumchlorid | 0,1 bis 6,0 | 0,2 bis 4,0 | 0,4 bis 2,0 | 0,5 | 0,5 |
| Vinylpyrrolidon/Vinylacetat-Copolymer | 0,5 bis 12 | 1,0 bis 10 | 2,0 bis 8,0 | 5,0 | 2,0 |
| Wasser | 70 bis 99 | 75 bis 95 | 80 bis 90 | 87 | 85 |
| Misc | add 100 | add 100 | add 100 | add 100 | add 100 |

| | Formel 71 | Formel 72 | Formel 73 | formel 74 | Formel 75 |
|---|---|---|---|---|---|
| Quaternisiertes Collagenhydrolysat a) | 0,05 bis 4,0 | 0,1 bis 2,0 | 0,2 bis 1,0 | 0,3 | 1,0 |
| Cetyltrimethylammoniumchlorid | 0,1 bis 6,0 | 0,2 bis 4,0 | 0,4 bis 2,0 | 0,5 | 0,5 |
| VinylpyrrolidonNinylacetat-Copolymer | 0,5 bis 12 | 1,0 bis 10 | 2,0 bis 8,0 | 5,0 | 2,0 |
| Wasser | 70 bis 99 | 75 bis 95 | 80 bis 90 | 87 | 85 |
| Ethanol | 1,0 bis 10 | 2,0 bis 8,0 | 3,0 bis 6,0 | 5,0 | 5,0 |
| Misc | add 100 | add 100 | add 100 | add 100 | add 100 |

| | Formel 76 | Formel 77 | Formel 78 | Formel 79 | Formel 80 |
|---|---|---|---|---|---|
| Quaternisiertes Collagenhydrolysat a) MW: 2000 und 2800 g/mol | 0,05 bis 4,0 | 0,1 bis 2,0 | 0,2 bis 1,0 | 0,3 | 1,0 |
| Cetyltrimethylammoniumchlorid | 0,1 bis 6,0 | 0,2 bis 4,0 | 0,4 bis 2,0 | 0,5 | 0,5 |
| Vinylpyrrolidon/Vinylacetat-Copolymer | 0,5 bis 12 | 1,0 bis 10 | 2,0 bis 8,0 | 5,0 | 2,0 |
| Wasser | 70 bis 99 | 75 bis 95 | 80 bis 90 | 87 | 85 |
| Ethanol | 1,0 bis 10 | 2,0 bis 8,0 | 3,0 bis 6,0 | 5,0 | 5,0 |
| Misc | add 100 | add 100 | add 100 | add 100 | add 100 |

| | Formel 111 | Formel 112 | Formel 113 | Formel 114 | Formel 115 |
|---|---|---|---|---|---|
| Quaternisiertes Collagenhydrolysat a) | 0,05 bis 4,0 | 0,1 bis 2,0 | 0,2 bis 1,0 | 0,3 | 1,0 |
| Cetyltrimethylammoniumchlorid | 0,1 bis 6,0 | 0,2 bis 4,0 | 0,4 bis 2,0 | 0,5 | 0,5 |
| Vinylpyrrolidon/Vinylacetat-Copolymer | 0,5 bis 12 | 1,0 bis 10 | 2,0 bis 8,0 | 5,0 | 2,0 |
| Seidenproteinhydrolysat | 0,05 bis 4,0 | 0,1 bis 2,0 | 0,2 bis 1,0 | 0,5 | 0,3 |
| Wasser | 70 bis 99 | 75 bis 95 | 80 bis 90 | 87 | 85 |
| Ethanol | 1,0 bis 10 | 2,0 bis 8,0 | 3,0 bis 6,0 | 5,0 | 5,0 |
| Misc | add 100 | add 100 | add 100 | add 100 | add 100 |

| | Formel 116 | Formel 117 | Formel 118 | Formel 119 | Formel 120 |
|---|---|---|---|---|---|
| Quaternisiertes Collagenhydrolysat a) MW: 2000 und 2800 g/mol | 0,05 bis 4,0 | 0,1 bis 2,0 | 0,2 bis 1,0 | 0,3 | 1,0 |
| Cetyltrimethylammoniumchlorid | 0,1 bis 6,0 | 0,2 bis 4,0 | 0,4 bis 2,0 | 0,5 | 0,5 |
| Vinylpyrrolidon/Vinylacetat-Copolymer | 0,5 bis 12 | 1,0 bis 10 | 2,0 bis 8,0 | 5,0 | 2,0 |
| Seidenproteinhydrolysat | 0,05 bis 4,0 | 0,1 bis 2,0 | 0,2 bis 1,0 | 0,5 | 0,3 |
| Wasser | 70 bis 99 | 75 bis 95 | 80 bis 90 | 87 | 85 |
| Ethanol | 1,0 bis 10 | 2,0 bis 8,0 | 3,0 bis 6,0 | 5,0 | 5,0 |
| Misc | add 100 | add 100 | add 100 | add 100 | add 100 |

Die erfindungsgemäßen kosmetischen Zusammensetzungen können weitere Hilfs-, Pflege- und Zusatzstoffe enthalten. Der Gewichtsanteil der in den erfindungsgemäßen Zusammensetzungen neben den Komponenten a) bis d) und den gegebenenfalls zugesetzten Lösungsmitteln enthaltenen weiteren Inhaltsstoffe, insbesondere der in diesen Zusammensetzungen enthaltenen weiteren Hilfs-, Pflege- und Zusatzstoffe am Gesamtgewicht der erfindungsgemäßen Zusammensetzungen beträgt vorzugsweise weniger als 10 Gew.-%, bevorzugt weniger als 5,0 Gew.-%, besonders bevorzugt weniger als 2,0 Gew.-% und insbesondere weniger als 1,0 Gew.-%. Der Gewichtsanteil dieser Hilfs-, Pflege- und Zusatzstoffe am Gesamtgewicht der erfindungsgemäßen kosmetischen Mittel kann beispielsweise 0,001 bis 2 Gew.-%, insbesondere 0,01 bis 0,5 Gew.-%, betragen.

Als weiteren Pflegestoff kann die erfindungsgemäße Zusammensetzung ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden.

Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Tierische Proteinhydrolysate sind beispielsweise Seiden-Proteinhydrolysate, die auch in Form von Salzen vorliegen können. Sie werden beispielsweise unter der Marke "Promois® (Interorgana)" vertrieben. Ein erfindungsgemäß bevorzugtes temperaturstabiles tierisches Proteinhydrolysat ist Promois Silk 1000. Pflanzliche Proteinhydrolysate sind beispielsweise Weizenproteinhydrolysate, welche erfindungsgemäß bevorzugte pflanzliche Proteinhydrolysate sind.

Die erfindungsgemäßen Haarbehandlungsmittel können in den üblichen Applikationsformen, wie z.B. als Sprühlösung, als Aerosolspray, als Schaum oder Schüttlotion formuliert werden. Erfindungsgemäß bevorzugt ist die Formulierung als Sprühlösung.

Wie eingangs beschrieben, eignen sich die erfindungsgemäßen kosmetischen Zusammensetzungen als Hitzeschutzmittel für keratinische Fasern, insbesondere menschliches Haar im Rahmen wärmeunterstützter Verformungsverfahren. Die Zusammensetzungen zeichnen sich durch eine verminderte Geruchsbildung und bewirken überraschenderweise darüber hinaus bei Glätteisenanwendungen eine gegenüber den aus dem Stand der Technik bekannten thermischen Stylingsprays deutlich verbesserte Haltbarkeit der Glättung. Gleiches gilt für deren Einsatz im Zusammenhang mit der Lockung von Haaren unter Verwendung eines Lockenstabs.

Ein weiterer Gegenstand der vorliegenden Anmeldung ist daher die Verwendung einer erfindungsgemäßen Zusammensetzung als Hitzeschutzmittel für keratinische Fasern, die im Rahmen eines Verformungsverfahrens einer Wärmebehandlung mit Temperaturen von 50°C bis 350°C, vorzugsweise von 80 bis 300°C, besonders bevorzugt von 120 bis 260 °C und insbesondere von 150 bis 220°C unterworfen werden.

Beansprucht wird weiterhin die Verwendung einer erfindungsgemäßen Zusammensetzung als Glättungsmittel für keratinische Fasern, die im Rahmen eines Verformungsverfahrens einer Wärmebehandlung mit Temperaturen von 50°C bis 350°C, vorzugsweise von 80 bis 300°C, besonders bevorzugt von 120 bis 260 °C und insbesondere von 150 bis 220°C unterworfen werden.

Ein Verfahren zur temporären Verformung keratinischer Fasern, vorzugsweise menschlicher Haare, in dessen Verlauf die keratinischen Fasern einer Wärmebehandlung mit Temperaturen von 50°C bis 350°C, vorzugsweise von 80 bis 300°C, besonders bevorzugt von 120 bis 260 °C und insbesondere von 150 bis 220°C unterworfen werden, dadurch gekennzeichnet, dass die keratinischen Fasern vor der Wärmebehandlung mit einer erfindungsgemäßen kosmetischen Zusammensetzung beaufschlagt werden ist ein weiterer Gegenstand der vorliegenden Anmeldung. Die Beaufschlagung der keratinischen Fasern erfolgt vorzugsweise durch Aufsprühen der erfindungsgemäßen Zusammensetzungen.

### Beispiele

Die folgenden Sprühlösungen wurden hergestellt (Angaben in Gew.-%):

| | **A** | **B** |
|---|---|---|
| VP/VA Copolymer¹⁾ | 5,0 | 5,0 |
| Gluadin WQ PP²⁾ | 1,0 | -- |
| Lamequat L³⁾ | -- | 1,0 |
| Natriumbenzoat | 0,1 | 0,1 |
| Genamin CTAC⁴⁾ | 0,5 | 0,5 |
| Wasser, entsalzt | 87,87 | 87,87 |
| Ethanol | 5,0 | 5,0 |
| Phosporsäure, 85% | 0,03 | 0,03 |
| Promois Silk 1000⁵⁾ | 0,5 | 0,5 |

| | | |
|---|---|---|
| ¹⁾ Vinylpyrrolidon-Vinylacetat-Copolymer, stabilisiert mit 0,05 Gew.-% Dodecyltrimethylammoniumchlorid; INCI-Bezeichnung: VP/VA Copolymer ²⁾ INCI-Bezeichnung Lauryldimonium Hydroxypropyl Hydrolyzed Wheat Protein (ca. 35 Gew.-% Aktivsubstanz in Wasser) ³⁾ INCI-Bezeichnung Lauryldimonium Hydroxypropyl Hydrolyzed Collagen (ca. 35 Gew.-% Aktivsubstanz in Wasser); ⁴⁾ Trimethylhexadecylammoniumchlorid (ca. 29 Gew.-% Aktivsubstanz in Wasser); INCI-Bezeichnung: Cetrimonium Chloride ⁵⁾ Seidenproteinhydrolysat; INCI-Bezeichnung: Hydrolyzed Silk | | |

Die erfindungsgemäße Zusammensetzung B zeichnet sich bei Einsatz in wärmeunterstüzten Haarverformungsverfahren gegenüber der Zusammensetzung A durch einen verbesserten Hitzeschutz sowie verbesserte kosmetische Eigenschaften aus.

## Patentansprüche

1. Kosmetische Zusammensetzung, enthaltend
a) mindestens eine erste quartäre Ammoniumverbingung aus der Gruppe der quaternisiertes Collagenhydrolysate;
b) Cetyltrimethylammoniumchlorid
c) mindestens ein filmbildendes Polymer aus der Gruppe der VinylpyrrolidonNinylacetat-Copolymere
d) Wasser.

2. Kosmetische Zusammensetzung nach Anspruch 1, **dadurch gekennzeichnet, dass** diese die quartäre Ammoniumverbindung a) bezogen auf ihr Gesamtgewicht in Mengen von 0,05 bis 4,0 Gew.-%, vorzugsweise von 0,1 bis 2,0 Gew.-% und inbesondere von 0,2 bis 1,0 Gew.-% enthält.

3. Kosmetische Zusammensetzung nach einem der vorherigen Ansprüche, **dadurch gekennzeichnet, dass** die quartäre Ammoniumverbingung a) ein Molekulargewicht oberhalb 1000 g/mol, vorzugsweise zwischen 1200 und 10000 g/mol, besonders bevorzugt zwischen 1500 und 5000 g/mol und insbesondere zwischen 2000 und 2800 g/mol aufweist.

4. Kosmetische Zusammensetzung nach einem der vorherigen Ansprüche, **dadurch gekennzeichnet, dass** diese die quartäre Ammoniumverbindung b) bezogen auf ihr Gesamtgewicht in Mengen von 0,1 bis 6,0 Gew.-%, vorzugsweise von 0,2 bis 4,0 Gew.-% und inbesondere von 0,4 bis 2,0 Gew.-% enthält.

5. Kosmetische Zusammensetzung nach einem der vorherigen Ansprüche, **dadurch gekennzeichnet, dass** diese das filmbildende Polymer c) bezogen auf ihr Gesamtgewicht in Mengen von 0,5 bis 12 Gew.-%, vorzugsweise von 1,0 bis 10 Gew.-% und inbesondere von 2,0 bis 8,0 Gew.-% enthält.

6. Kosmetische Zusammensetzung nach einem der vorherigen Ansprüche, **dadurch gekennzeichnet, dass** das kosmetische Mittel, bezogen auf sein Gesamtgewicht 70 bis 99 Gew.-%, vorzugweise 75 bis 95 Gew.-% und insbesondere 80 bis 90 Gew.-% Wasser enthält.

7. Verwendung einer Zusammensetzung nach einem der vorherigen Ansprüche als Hitzeschutzmittel für keratinische Fasern, die im Rahmen eines Verformungsverfahrens einer Wärmebehandlung mit Temperaturen von 50°C bis 350°C unterworfen werden.

8. Verfahren zur temporären Verformung keratinischer Fasern, vorzugsweise menschlicher Haare, in dessen Verlauf die keratinischen Fasern einer Wärmebehandlung mit Temperaturen von 50°C bis 350°C unterworfen werden, **dadurch gekennzeichnet, dass** die keratinischen Fasern vor der Wärmebehandlung mit einer kosmetischen Zusammensetzung nach einem der Ansprüche 1 bis 6 beaufschlagt werden.

## Claims

1. A cosmetic composition, containing
a) at least one first quaternary ammonium compound from the group of quaternized collagen hydrolysates;
b) cetyl trimethyl ammonium chloride;
c) at least one film-forming polymer from the group of vinylpyrrolidone/vinyl acetate copolymers;
d) water.

2. The cosmetic composition according to claim 1, **characterized in that** it contains the quaternary ammonium compound a) in quantities of from 0.05 to 4.0 wt.%, preferably from 0.1 to 2.0 wt.%, and in particular from 0.2 to 1.0 wt.%, based on the total weight of the composition.

3. The cosmetic composition according to either of the preceding claims, **characterized in that** the quaternary ammonium compound a) has a molecular weight of above 1,000 g/mol, preferably between 1,200 and 10,000 g/mol, particularly preferably between 1,500 and 5,000 g/mol, and in particular between 2,000 and 2,800 g/mol.

4. The cosmetic composition according to one of the preceding claims, **characterized in that** it contains the quaternary ammonium compound b) in quantities of from 0.1 to 6.0 wt.%, preferably from 0.2 to 4.0 wt.%, and in particular from 0.4 to 2.0 wt.%, based on the total weight of the composition.

5. The cosmetic composition according to one of the preceding claims, **characterized in that** it contains the film-forming polymer c) in quantities of from 0.5 to 12 wt.%, preferably from 1.0 to 10 wt.%, and in particular from 2.0 to 8.0 wt.%, based on the total weight of the composition.

6. The cosmetic composition according to one of the preceding claims, **characterized in that** the cosmetic agent contains from 70 to 99 wt.%, preferably from 75 to 95 wt.%, and in particular from 80 to 90 wt.%, of water, based on the total weight of the agent.

7. The use of a composition according to one of the preceding claims as a heat protection agent for keratin fibers which are subject to a heat treatment at temperatures of from 50 °C to 350 °C during a deformation method.

8. A method for temporarily deforming keratin fibers, preferably human hair, during the course of which the keratin fibers are subject to a heat treatment at temperatures of from 50 °C to 350 °C, **characterized in that** a cosmetic composition according to one of claims 1 to 6 is applied to the keratin fibers before the heat treatment.

## Revendications

1. Composition cosmétique contenant :
a) au moins un premier composé d'ammonium quaternaire issu du groupe des hydrolysats de collagène quaternaires ;
b) du chlorure de cétyltriméthylammonium ;
c) au moins un polymère filmogène issu du groupe des copolymères de vinylpyrrolidone/d'acétate de vinyle ;
d) de l'eau.

2. Composition cosmétique selon la revendication 1, **caractérisée en ce que** celle-ci contient le composé d'ammonium quaternaire a), rapporté à son poids total, dans des quantités allant de 0,05 à 4,0 % en poids, de préférence de 0,1 à 2,0 % en poids et en particulier de 0,2 à 1,0 % en poids.

3. Composition cosmétique selon l'une des revendications précédentes, **caractérisée en ce que** le composé d'ammonium quaternaire a) présente un poids moléculaire supérieur à 1 000 g/mol, de préférence entre 1 200 et 10 000 g/mol, de manière particulièrement préférée entre 1 500 et 5 000 g/mol et en particulier entre 2 000 et 2 800 g/mol.

4. Composition cosmétique selon l'une des revendications précédentes, **caractérisée en ce que** celle-ci contient le composé d'ammonium quaternaire b), rapporté à son poids total, dans des quantités allant de 0,1 à 6,0 % en poids, de préférence de 0,2 à 4,0 % en poids et en particulier de 0,4 à 2,0 % en poids.

5. Composition cosmétique selon l'une des revendications précédentes, **caractérisée en ce que** celle-ci contient le polymère filmogène c), rapporté à son poids total, dans des quantités allant de 0,5 à 12 % en poids, de préférence de 1,0 à 10 % en poids et en particulier de 2,0 à 8,0 % en poids.

6. Composition cosmétique selon l'une des revendications précédentes, **caractérisée en ce que** l'agent cosmétique, rapporté à son poids total, contient de 70 à 99 % en poids, de préférence de 75 à 95 % en poids et en particulier de 80 à 90 % en poids en eau.

7. Utilisation d'une composition selon l'une des revendications précédentes en tant qu'agent de protection thermique pour fibres kératiniques, qui sont soumises à un traitement thermique comportant des températures allant de 50 °C à 350 °C dans le cadre d'un procédé de mise en forme.

8. Procédé de mise en forme temporaire de fibres kératiniques, de préférence des cheveux humains, au cours duquel les fibres kératiniques sont soumises à un traitement thermique comportant des températures allant de 50 °C à 350 °C, **caractérisé en ce que** l'on applique une composition cosmétique selon l'une des revendications 1 à 6 sur les fibres kératiniques avant le traitement thermique.
